# EUROPEAN PATENT APPLICATION

(11) **EP 3 782 594 A1**
(43) Date of publication of application: **24.02.2021**
(21) Application number: 19195444.5
(22) Date of filing: 04.09.2019
(51) Int. Cl.: A61F 13/26

(54) **CATHETER TAMPON AND TELESCOPIC CATHETER SET AND FINGER SHEATH THEREOF**

(30) Priority: 21.08.2019 WO PCT/CN2019/101829
(71) Applicant: Wu, Ching-Ju, Kaohsiung City (TW)
(72) Inventor: Wu, Ching-Ju, Kaohsiung City (TW)
(74) Representative: Lang, Christian

(57) **Abstract**

Provided is a catheter tampon and a telescopic catheter set and a finger set thereof. The telescopic catheter set includes an outer tube (1) having a sheet (11, 51) partially overlapped by curling, with a chamber (**S**) formed inside the sheet (11, 51). An inner edge (11a, 51a) of the sheet (11, 51) is not exposed and an outer edge (11b, 51b) of the sheet (11, 51) is exposed. An anterior edge (11c, 51c) of the sheet (11, 51) circles to form an opening (12, 52), and a posterior edge (11d, 51d) of the sheet (11, 51) circles to form an entrance (13, 53). The telescopic catheter set further includes an inner tube (2) having a first end (21) inside the chamber (**S**) and movable relative to the outer tube (1), and a second end (22) at the posterior edge (11d, 51d) of the entrance (13, 53).

## Description

### BACKGROUND OF THE INVENTION

### 1. Field of the Invention

The present invention relates to a female sanitary article and components thereof and, more particularly, to a catheter tampon put into a user's body for absorbing the menstrual blood, and a telescopic catheter and a finger sheath of the catheter tampon.

### 2. Description of the Related Art

Common catheter tampons generally include an insertion tube, a tampon and a push tube. The insertion tube is inserted into a female vagina. The front end of the insertion tube is in a hemispherical shape with a petal-like opening. The tampon locates inside the insertion tube. The push tube is pushed from a back end of the insertion tube, allowing the petal-like opening shoved by the tampon to be located in an appropriate position in the female vagina. The insertion tube and the push tube may be discarded. However, both insertion tube and push tube are plastic products, thus an environmental issue is raised though it is convenient to use the tampon. In addition, the front end of the insertion tube becomes rough as forming the petal-like opening in an improper surface treatment, resulting in causing friction, discomfort, and even puncture wound and scratch wound during the process of insertion of the insertion tube into the vagina by a user.

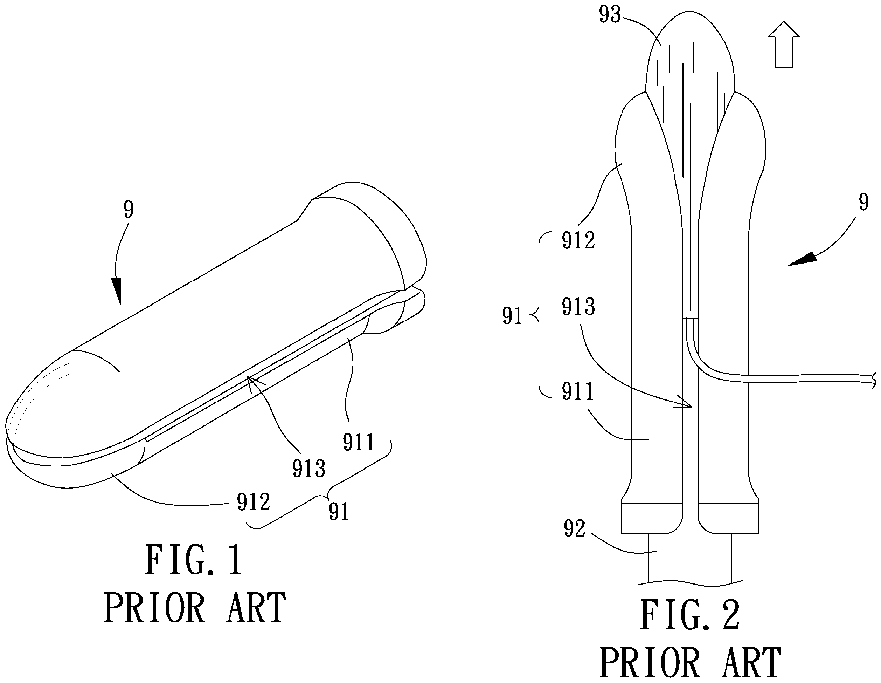

Referring to FIGS. 1 and 2, a conventional tampon instrument 9 is shown. The conventional tampon instrument 9 includes a reusable cylindrical body 91 and a pusher 92. The cylindrical body 91 has a lateral wall 911 connected to a hemispherical head 912, a ditch 913 which half splits the head 912 from one side of the lateral wall 911 to the end. As using the conventional tampon instrument 9, the cylindrical body 91 may be opened from a lateral side of the cylindrical body 91 along the ditch 913, allowing the tampon 93 to be put into the cylindrical body 91. The tampon 93 may be pushed out of the cylindrical body 91 and be put into the female vagina by pushing the pusher 92. One embodiment similar to the conventional tampon instrument 9 is disclosed in China patent application No. 108670560.

The hemispherical head 912 of the conventional tampon instrument 9 is half split by the ditch 913, and thus the head 912 would be expanded outward along two lateral sides of the ditch 913 as the cylindrical body 91 is opened by the tampon 93. However, a front end of the head 912 will generate a clamping force from the two lateral sides of the ditch 913 to clamp the tampon 93 inward, which makes harder for the tampon 93 to be pushed out. In addition, the ditch 913 will also be expanded around the lateral wall 911 when the head 912 is expanded by the tampon 93. As a result, the ditch 913 may pinch the vaginal wall of the user when the lateral wall 911 is returning to the original position.

Thus, a conventional catheter set for assisting in putting the tampon into female body is necessary to be improved.

### SUMMARY OF THE INVENTION

To solve the problems mentioned above, the purpose of the present invention is to provide a catheter tampon and a telescopic catheter set and finger sheath thereof. The front end of the outer tube does not have a petal-like opening, and the opening of the outer tube at a front end is uniformly expanded without generating a resistance for clamping the tampon as pushing the tampon.

Another purpose of the present invention is to provide a catheter and a telescopic catheter set and a finger sheath thereof, which are easy to be manufactured and assembled.

Another purpose of the present invention is to provide a catheter and a telescopic catheter set and a finger sheath thereof, which provides good comfortability of use without puncturing or pinching vaginal wall during insertion.

Another purpose of the present invention is to provide a catheter and a telescopic catheter set and a finger sheath thereof, with a degradable material or a reusable material after washing to meet the requirement of environmental protection concept.

In the various figures of the drawings, the same numerals designate the same or similar parts. Furthermore, when the terms "front", "rear", "left", "right", "up (top)", "down (bottom)", "inner", "outer", "lateral side", and similar terms are used hereinafter, it should be understood that these terms have reference only to the structure shown in the drawings as it would appear to a person viewing the drawings, and are utilized only to facilitate describing the invention.

"A" or "an" used in the elements and components recited in the disclosure of the present invention is for convenient use and provides generally meanings of the scope of the present invention. "A" or "an" used in the present invention is interpreted as comprising one or at least one, and the single concept also includes plural conditions, otherwise obviously indicating other meanings.

"Connection", "combination" or "assembling" and similar terms described in the full text of the present invention mainly include types of the components detached without destroying the components after connecting, or components which are inseparable after connecting, which may be chose by a person ordinarily skilled in the art according to the component material for assembling or assembly requirements.

A telescopic catheter set of the present invention includes an outer tube having a sheet partially overlapped by curling. A chamber is formed inside the sheet, with an inner edge of the sheet not exposed and an outer edge of the sheet exposed. An anterior edge of the sheet circles to form an opening, and a posterior edge of the sheet circles to form an entrance. The telescopic catheter set further includes an inner tube, with a first end of the inner tube located inside the chamber and being movable relative to the outer tube, and with a second end of the inner tube located at the posterior edge of the entrance.

A catheter tampon of the present invention includes a telescopic catheter set mentioned above and a tampon. The tampon has an absorption body inside the chamber, and a string positioned at end of the absorption body, with the string passing through the inner tube and through out of a second end of the inner tube.

A finger sheath of tampon of the present invention has a sheet partially overlapped by curling. A chamber is formed inside the sheet, with an inner edge of the sheet not exposed and an outer edge of the sheet exposed. An anterior edge of the sheet circles to form an opening, and a posterior edge of the sheet circles to form an entrance.

Accordingly, a catheter tampon and a telescopic catheter set and a finger sheath thereof may allow, by curling the outer tube (or the finger sheath of the tampon) to a partially overlapped type, the opening of the outer tube (or the finger sheath of the tampon) at a front end to be uniformly expanded without generating a resistance for clamping the tampon as pushing the tampon. In addition, it is not necessary to dispose a peta-like opening at a front end of the outer tube (or the finger sheath of the tampon), allowing the structure of the outer tube (or the finger sheath of the tampon) to be simplified for forming, and the absorption body of the tampon to be loaded from a lateral side of the outer tube (or the finger sheath of the tampon). It provides effects of enhancing operational convenience, decreasing manufacturing cost and enhancing manufacturing and assembly convenience.

In an example, the anterior edge of the sheet is shorter than the posterior edge of the sheet, and wherein the outer edge of the sheet tiltedly connects to the anterior edge and the posterior edge of the sheet. As such, less areas are overlapped adjacent to the opening of the sheet. It may decrease the resistance as the opening is expanded, and provides effects of enhancing operational convenience.

In an example, the outer edge of the sheet forms a round corner adjacent to the posterior edge of the sheet. As such, it may enhance the smoothness of the outer surface of the outer tube, decrease the uncomfortability of the user as inserting the outer tube into the vagina, and provide the effect of enhancing comfortability of use.

In an example, the outer tube has an anti-slip portion positioned on an exposed surface of the sheet. The anti-slip portion is adjacent to the entrance and is away from the opening. As such, it is easy to form the anti-slip portion. In addition, a user may hold the anti-slip portion, ensuring the outer tube not to be slipped off. It may provide the effects of enhancing manufacturing convenience and operational convenience.

In an example, the sheet is made of degradable materials. As such, it may meet the requirement of environmental protection concept.

In an example, the inner tube has a longitudinal ditch extended from the first end to the second end. As such, a strip of the tampon may be put inside of the inner tube from the lateral side thereof. It may further enhance the effects of manufacturing convenience and efficiency.

In an example, the inner tube is crimped and thermoformed from a plate, and the longitudinal ditch is formed between a first lateral edge and a second lateral edge opposite to the first lateral edge of the plate. As such, it may provide the effect of enhancing manufacturing convenience.

In an example, the telescopic catheter set further includes a connection belt. A first end of the connection belt connects to the inner edge of the sheet, and a second end of the connection belt connects to the first lateral edge of the plate. The connection belt is coiled around a periphery of the inner tube inside the chamber. As such, a shape of the telescopic catheter is easy to be formed in once curling process, followed by thermoforming. It may provide the effect of enhancing manufacturing process. In addition, the connection belt may restrict the displacement amplitude of the inner tube, ensuring the inner tube not to drop into the vagina. It may also simplify the structures of the outer tube and the inner tube, and provide the effects of enhancing use safety and decreasing manufacturing cost.

In an example, the first end of the connection belt may be adjacent to a posterior edge of the sheet, and the second of the connection belt may be adjacent to an anterior end of the plate. As such, it may ensure that the action of the inner tube would not be affected by the connection belt, providing the effect of enhancing operational convenience.

As to the finger sheath of tampon, the sheet is made of degradable materials. As such, it may meet the requirement of environmental protection concept.

The finger sheath of tampon is formed in a conical tubular shape, with the entrance larger than the opening. As such, it is easy for different users to put fingers into the chamber, providing the effect of enhancing usability for different users.

### BRIEF DESCRIPTION OF THE DRAWINGS

The present invention will become more fully understood from the detailed description given hereinafter and the accompanying drawings which are given by way of illustration only, and thus are not limitative of the present invention, and wherein:
FIG. 1 shows a stereoscopic view of a conical body of a conventional tampon instrument.
FIG. 2 is a usage state of a conventional tampon instrument.
FIG. 3 is an expanded view of an outer tube of a telescopic catheter set according to the first embodiment of the present invention.
FIG. 4 is a stereoscopic exploded view of an outer tube partially opened of a telescopic catheter set according to the first embodiment of the present invention.
FIG. 5 is a stereoscopic view of a telescopic catheter set according to the first embodiment of the present invention.
FIG. 6 is a cross sectional view along line A-A of FIG. 5.
FIG. 7 is a usage state of a telescopic catheter set according to the first embodiment of the present invention.
FIG. 8 is a cross sectional view along line B-B of FIG. 7.
FIG. 9 is a stereoscopic view of a telescopic catheter set according to the second embodiment of the present invention.
FIG. 10 is an expanded view of a telescopic catheter set according to the third embodiment of the present invention.
FIG. 11 is a stereoscopic view of a telescopic catheter set according to the third embodiment of the present invention.
FIG. 12 is a cross sectional view along line C-C of FIG. 11.
FIG. 13 is a usage state of a telescopic catheter set according to the third embodiment of the present invention.
FIG. 14 is a stereoscopic view of a telescopic catheter set according to the fourth embodiment of the present invention.
FIG. 15 is an expanded view of a finger sheath of tampon of the present invention.
FIG. 16 is a stereoscopic view of a finger sheath of tampon partially opened of the present invention.
FIG. 17 is a usage state of a finger sheath of tampon of the present invention.

### DETAILED DESCRIPTION OF THE INVENTION

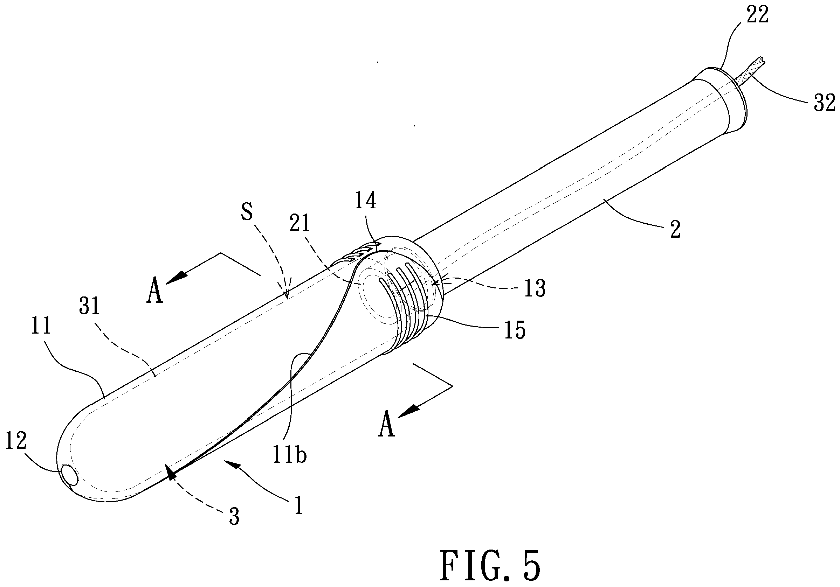

Referring to FIG. 5, a telescopic catheter set of the first embodiment of the present invention incudes an outer tube 1 and an inner tube 2. One end of the inner tube 2 is inserted into the outer tube 1 and is movable relative to the outer tube 1.

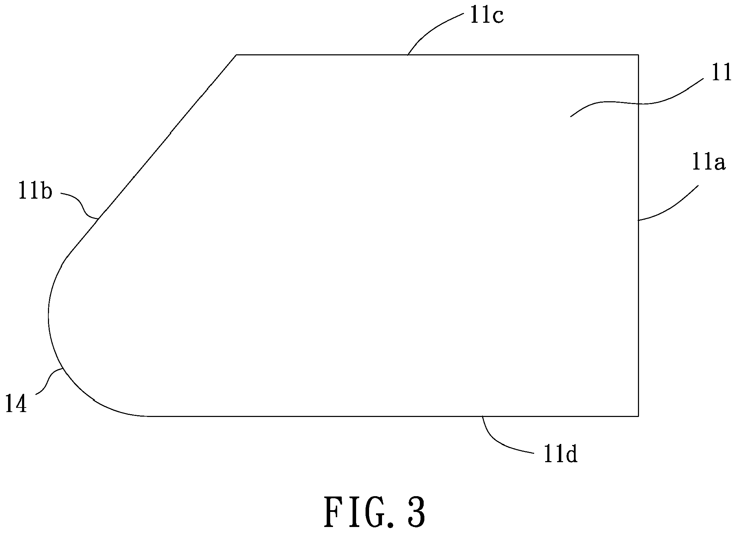

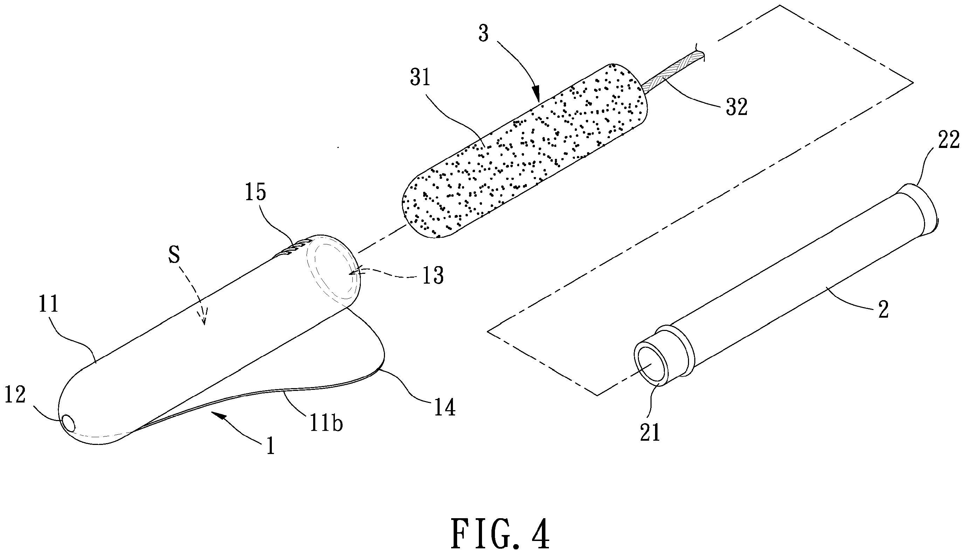

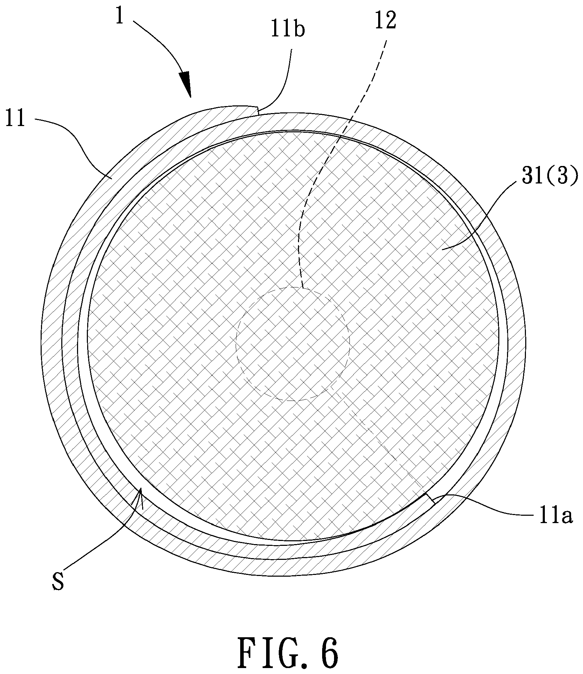

Referring to FIGS. 3 and 4, the outer tube 1 has a sheet 11. The sheet 11 is partially overlapped by curling (referring to FIG. 6) and a chamber S is formed inside the sheet 11, making an inner edge 11a of the sheet 11 not exposed and an outer edge 11b of the sheet 11 exposed. In addition, an anterior edge 11c of the sheet 11 circles to form an opening 12, and wherein a posterior edge 11d of the sheet 11 circles to form an entrance 13.

In the present embodiment, the anterior edge 11c of the sheet 11 may be shorter than posterior edge 11d of the sheet 11, allowing the outer edge 11b of the sheet 11 to be tiltedly connected to the anterior edge 11c and the posterior edge 11d of the sheet 11. As such, less areas are overlapped adjacent to the opening 12 of the sheet 11 to decrease the resistance as the opening 12 is expanded (detail described below). In addition, the sheet 11 of the present embodiment may form a round corner 14 at a junction between the outer edge 11b and the posterior edge 11d, or from a type of the outer edge 11b having a thickness gradually thinner. It may enhance the smoothness of the outer surface of the outer tube 1 to decrease the uncomfortability of the user as inserting the outer tube 1 into the vagina.

In addition, for example but not limited to, the outer tube 1 may be formed in a tubular shape by curling a planar shaped sheet 11, and shaped by thermoforming based on the materials or other methods. The front end of the outer tube 1 is in a hemispherical shape and has the opening 12 in a circle shape. Aback of the outer tube 1 may be shaped into an aspect of having diameter gradually decreased. In addition, as thermoforming the outer tube 1, an anti-slip portion 15 may be formed on an exposed surface of the sheet 11. The anti-slip portion 15 may be ribs, bumps or other structures having the similar function, ensuring the outer tube 1 not to be slipped off when being held by a user. Preferably, the anti-slip portion 15 is adjacent to the entrance 13 and is away from the opening 12.

A first end 21 of the inner tube 2 is located inside the chamber S and is axially movable relative to the outer tube 1, and a second end 22 of the inner tube 2 is located at the posterior edge of the entrance 13.

According to the structure mentioned above, as an absorption body 31 of a tampon 3 is put inside the chamber S of the outer tube 1 of the telescopic catheter set of the present embodiment, a string 32 positioned at an end of the absorption body 31 may pass through the inner tube 2 and through out of a second end 22 of the inner tube 2.

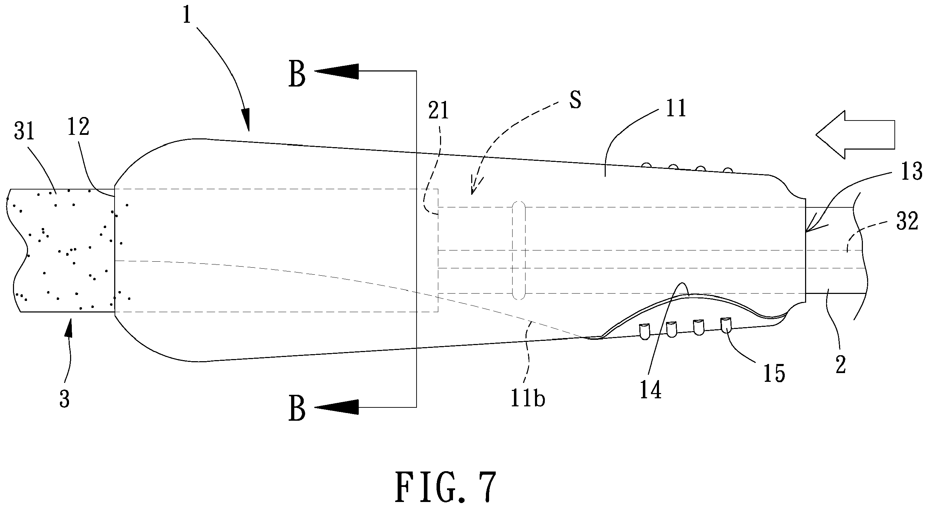

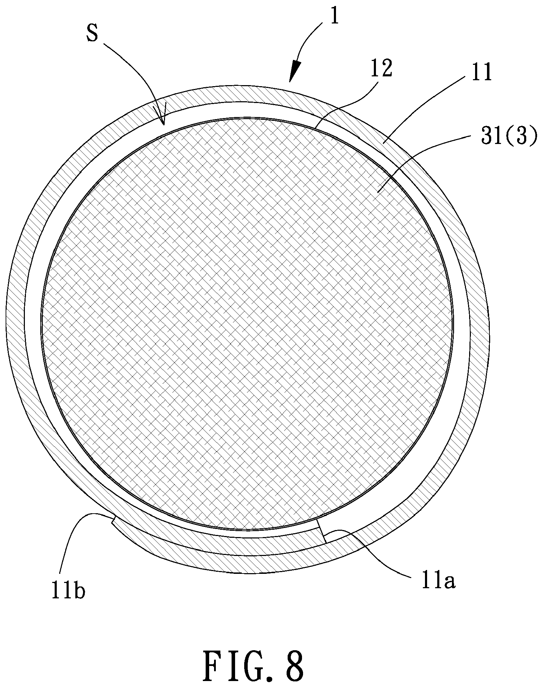

Referring to FIGS. 7 and 8, when using the telescopic catheter set, the user may pinch the anti-slip portion 15 of the outer tube 1, and insert a front end of the outer tube 1 into a vagina of the user, followed by pushing the second end 22 of the inner tube 2 to allow the absorption body 31 to move forward by the pushing of the first end 21 of the inner tube 2. As the absorption body 31 touches the inner surface of the front end of the outer tube 1, the sheet 11 may gradually expand along with the forward movement of the absorption body 31 by the friction. It allows the opening 12 of the outer tube 1 to be expanded uniformly, and thus the absorption body 31 may be pushed out by the inner tube 2, till entire detachment of the outer tube 1 and positioned in a proper depth of the vagina. Subsequently, the user may pull out the outer tube 1, allowing the inner tube 2 to be discarded along with the outer tube 1 from the body. Part of the string 32 of the tampon 3 is exposed outside the body, so that the absorption body 31 may be pulled out of the body through the string 32.

Referring to FIG. 5, it is noted that the telescopic catheter set of the present invention may not only be suitable for disposable catheter tampons, but also be suitable for reusable tampons to meet the requirement of environmental protection concept.

In detail, as applying to the catheter tampons, an absorption body 31 of the tampon 3 may be preloaded into the outer tube 1 during the production and the string 32 of the tampon 3 is allowed to be passed through the second end 22 of the inner tube 2. Each catheter tampon is packaged individually. For the telescopic catheter set applied to the catheter tampons, the outer tube 1 and the inner tube 2 are preferably made of degradable materials such as paper or biodegradable material, allowing the catheter tampon to meet the requirement of convenience and environmental protection.

When used as tampon instrument, the outer tube 1 and the inner tube 2 may be made of water resistant materials. When using the tampon instrument, an absorption body 31 of a tampon 3 is loaded into the outer tube 1 from a lateral side of the outer tube 1 by opening the curled outer tube 1, allowing a string 32 of the tampon 3 to be passed through the inner tube 2. As the tampon 3 is put into the vagina in a proper depth, the outer tube 1 and the inner tube 2 taken out of the vagina may be reused after washing.

For the reason that the absorption body 31 of the tampon 3 of the telescopic catheter set of the present invention may be loaded into the outer tube 1 from the lateral side of the outer tube 1 through the curled shape of the outer tube 1, the absorption body 31 of the tampon 3 may be loaded into the outer tube 1 quickly, to enhance the packaging efficiency or usage assembly efficiency.

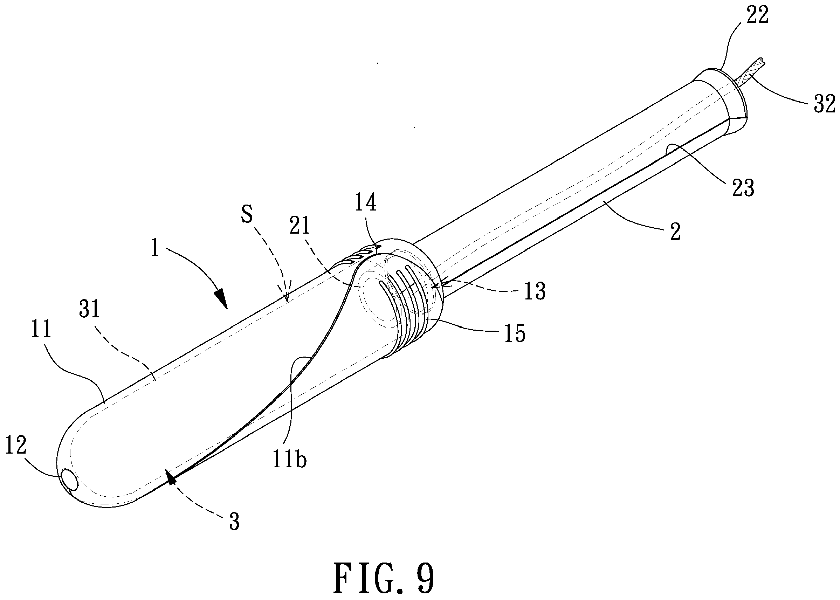

Referring to FIG. 9, it shows a second embodiment of the telescopic catheter set of the present invention. In the present embodiment, the inner tube 2 further has a longitudinal ditch 23 extended from the first end 21 to the second end 22. As such, not only the absorption body 31 of the tampon 3 may be loaded into the outer tube 1 from the lateral side of the outer tube 1, but also the string 32 of the tampon 3 may be loaded into the inner tube 2 through the longitudinal ditch 23, to further enhance the assembly convenience and efficiency.

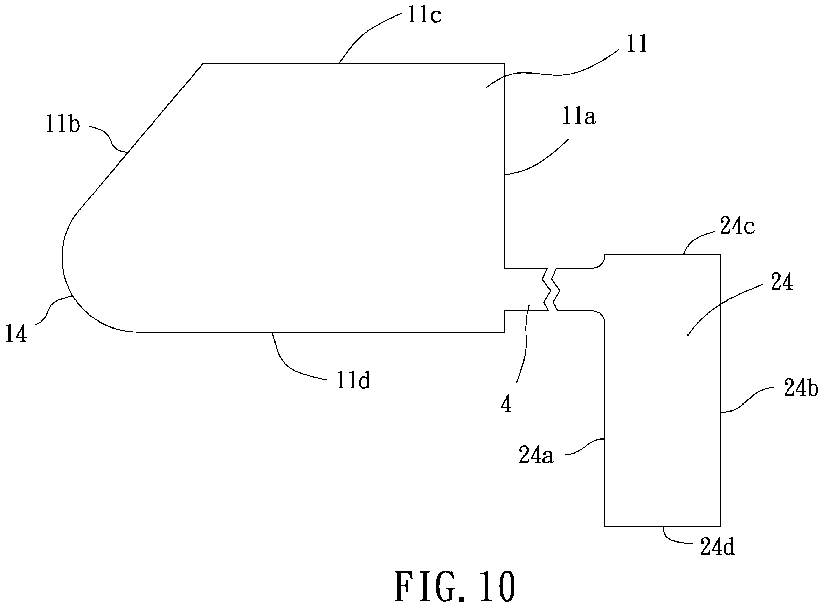

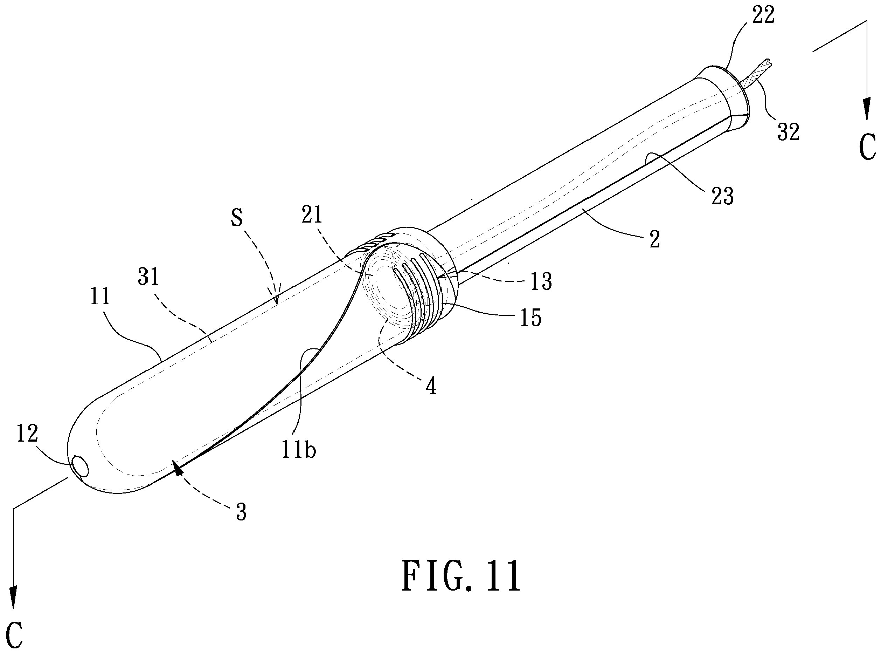

Referring to FIGS. 10 and 11, a third embodiment of the telescopic catheter set of the present invention is shown. In the present embodiment, the sheet 11 may be in a planar shape before thermoforming the outer tube 1, and the inner tube 2 may also be thermoformed by curling a plate 24. Therefore, the telescopic catheter set preferably further includes a connection belt 4. Two ends of the connection belt 4 connect to the sheet 11 and the plate 24, respectively, and the connection belt 4 is coiled around a periphery of the inner tube 2 inside the chamber S. In detail, a first end of the connection belt 4 may connect to the inner edge 11a of the sheet 11 and is positioned adjacent to a posterior edge 11d of the sheet 11. The second of the connection belt 4 connects to a first lateral edge 24a of the plate 24 and is adjacent to an anterior end 24c of the plate 24. After curling and thermoforming the plate 24, the longitudinal ditch 23 may be formed between the first lateral edge 24a and the second lateral edge 24b opposite to the first lateral edge 24a of the plate 24. After curling the plate 24, keeping curling the connection belt 4, allowing the connection belt 4 to be concentrically coiled with several turns around a periphery of the inner tube 2 followed by curling the sheet 11 to form the outer tube 1. Thus, the structure is convenient to form the telescopic catheter set by once curling process, followed by fixing the shape by thermoforming method based on materials or by other methods. It may provide the effect for enhancing manufacturing efficiency.

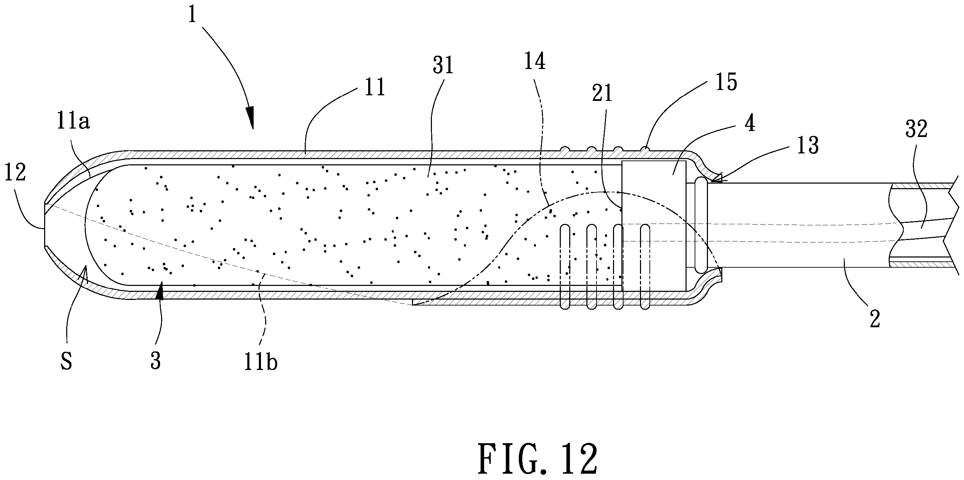

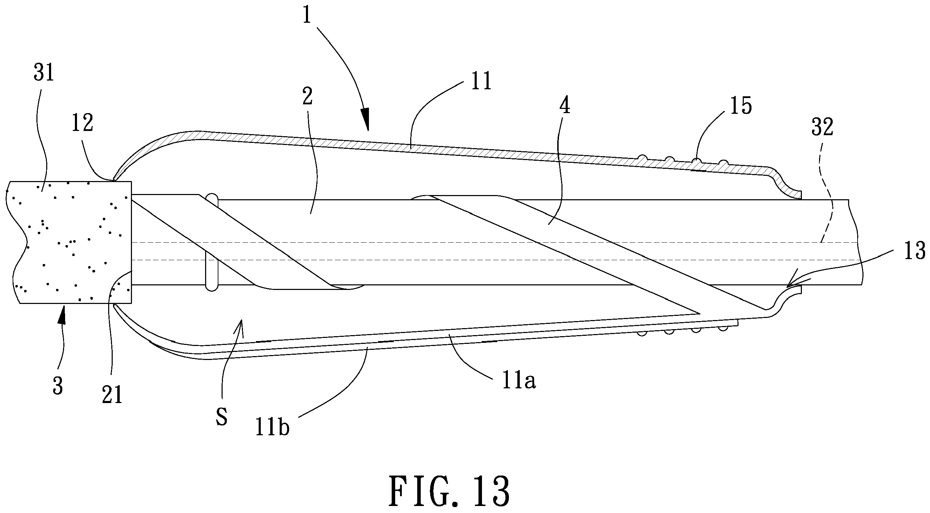

Referring to FIGS. 12 and 13, according to the structure mentioned above, when the absorption body 31 of the tampon 3 is positioned in the chamber S of the outer tube 1, the absorption body 31 is positioned in front of the connection belt 4. During the process of pushing the second end 22 of the inner tube 2 by the user and allowing the absorption body 31 to be moved forward by the pushing of the first end 21 of the inner tube 2, the connection belt 4 may be extended without affecting the action of the inner tube 2, and the connection belt 4 may also restrict the displacement amplitude of the inner tube 2. That is, even there is no any stop element positioned at the front end of the outer tube 1 or the inner tube 2, the inner tube 2 may be pulled to stop by the connection belt 4 as the connection belt 4 reaches at maximum extension. It prevents the inner tube 2 from falling into the vagina due to the operational error. In addition, the structures of the outer tube 1 and the inner tube 2 may be simplified accordingly.

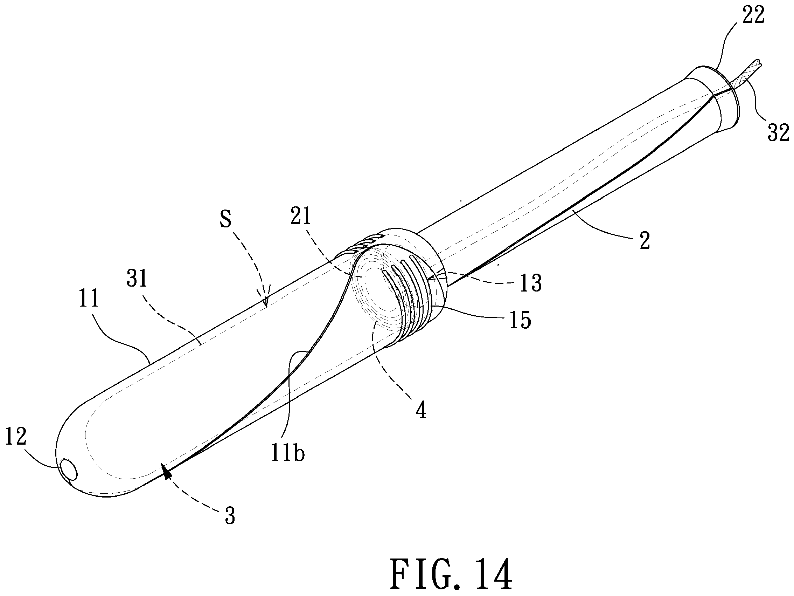

Referring to FIG. 14, it is a fourth embodiment of the telescopic catheter set of the present invention. In the present embodiment, the inner tube 2 may be similar to the outer tube 1, allowing the plate 24 to be curled into a partially overlapped form. A first lateral edge 24a of the plate 24 is not exposed and a second lateral edge 24b of the plate 24 is exposed. As such, the tolerance of the inner tube 2 thermoformed is large. It may provide the effect of enhancing manufacturing convenience.

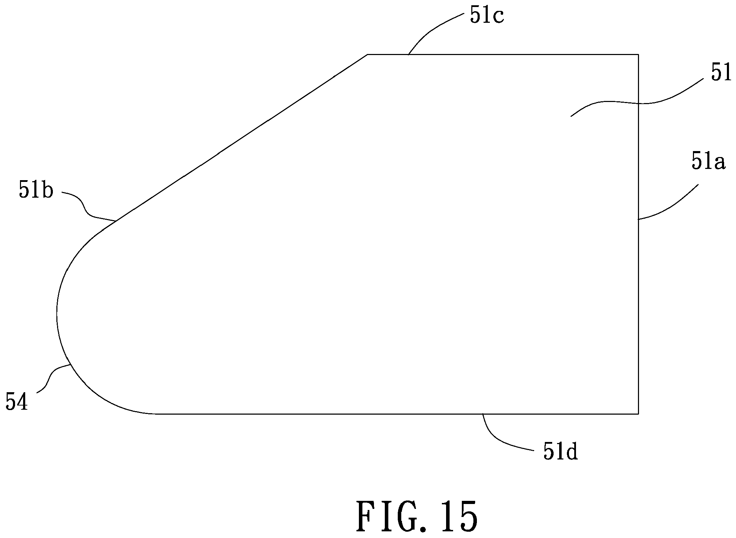

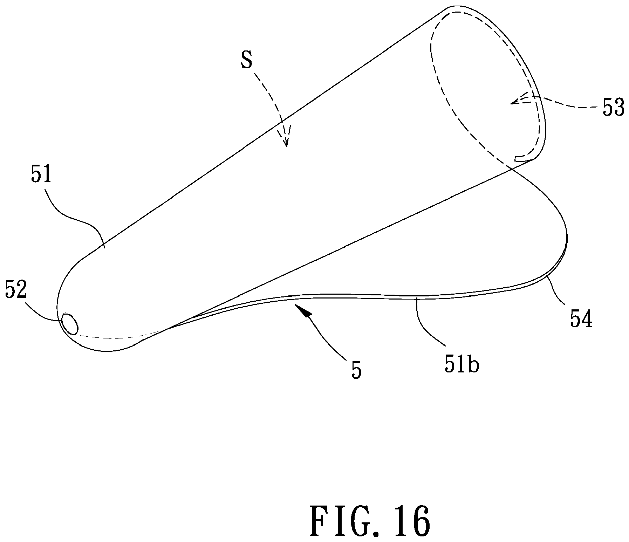

Referring to FIGS. 15 and 16, the present invention further provides a finger sheath of the tampon 5 that has a sheet 51 partially overlapped by curling. A chamber S is formed inside the sheet 51, and an inner edge 51a of the sheet 51 is not exposed and an outer edge 51b of the sheet 51 is exposed. An anterior edge 51c of the sheet 51 circles to form an opening 52, and a posterior edge 51d of the sheet 51 circles to form an entrance 53. The finger sheath of tampon 5 is preferably formed in a conical tubular shape, and the entrance 53 is larger than the opening 52, allowing fingers of different users to successfully pass the chamber S from the entrance 53.

In the present embodiment, it can be chosen to have the anterior edge 51c of the sheet 51 shorter than the posterior edge 51d of the sheet 51, and the outer edge 51b of the sheet 51 tiltedly connected to the anterior edge 51c and the posterior edge 51d of the sheet 11, which allows less areas to be overlapped adjacent to the opening 52 of the sheet 51. In addition, in the sheet 51 of the present embodiment, a round corner 54 may be formed at a junction between the outer edge 51b and the posterior edge 51d, or a type of the outer edge 11b having a thickness gradually thinner may be formed.

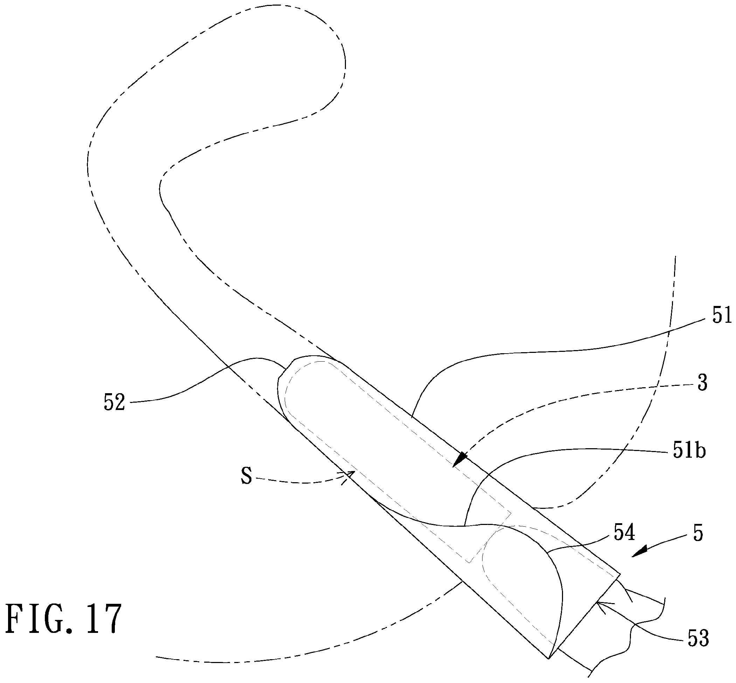

Referring to FIG. 17, as using the telescopic catheter set, the absorption body 31 of the tampon 3 may be loaded into the chamber S from the entrance 53, or from the lateral direction by opening the finger sheath of the tampon 5. Then, the finger of the user may be put in the finger sheath of the tampon 5 from the entrance 53, followed by inserting the front end of the finger sheath of the tampon 5 into the vagina. The back end of the finger sheath of the tampon 5 remains outside of the body. The absorption body 31 is subsequently moved forward by the finger, allowing the sheet 51 to be expanded gradually by the action of the absorption body 31. The absorption body 31 may be successfully pushed into a proper position in the vagina, and the finger sheath of the tampon 5 may be pulled out as the finger is pulled out of the body.

It is worth mentioning that the finger sheath of the tampon 5 of the present invention may be made by degradable materials such as paper or biodegradable materials. Thus, it may meet the requirement of environmental protection concept even it is disposable. Particularly, the structure of the finger sheath of the tampon 5 of the present invention is simple and easy to be opened for washing. Thus, it is preferably made of water resistant materials for reusing after washing.

Based the above, the catheter tampon and the telescopic catheter set and the finger sheath thereof may allow, by curling the outer tube (or the finger sheath of the tampon) to a partially overlapped type, the opening of the outer tube (or the finger sheath of the tampon) at a front end to be uniformly expanded without generating a resistance for clamping the tampon as pushing the tampon. In addition, it is not necessary to dispose a peta-like opening at a front end of the outer tube (or the finger sheath of the tampon), allowing the structure of the outer tube (or the finger sheath of the tampon) to be simplified for forming, and the puncture wound and scratch wound not to be caused in an improper surface treatment. Thus, the catheter tampon and the telescopic catheter set and the finger sheath thereof may provide the effects of enhancing operational convenience and decreasing the manufacturing cost.

In addition, the catheter tampon and the telescopic catheter set and the finger sheath thereof may allow the absorption body of the tampon to be loaded from a lateral side of the outer tube (or the finger sheath of the tampon) by making the outer tube (or the finger sheath of the tampon) partially overlapped through curling. It provides effects of enhancing manufacturing convenience and assembly convenient.

## Claims

1. A finger sheath of tampon, **characterized in** comprising a sheet (11, 51) partially overlapped by curling, with a chamber (S) formed inside the sheet (11, 51), wherein an inner edge (11a, 51a) of the sheet (11, 51) is not exposed and an outer edge (11b, 51b) of the sheet (11, 51) is exposed, wherein an anterior edge (11c, 51c) of the sheet (11, 51) circles to form an opening (12, 52), and wherein a posterior edge (11d, 51d) of the sheet (11, 51) circles to form an entrance (13, 53).

2. The finger sheath of tampon as claimed in claim 1, **characterized in that** the anterior edge (11c, 51c) of the sheet (11, 51) is shorter than the posterior (11d) edge of the sheet (11, 51), and wherein the outer edge (11b, 51b) of the sheet (11, 51) tiltedly connects to the anterior edge (11c, 51c) and the posterior edge (11d, 51d) of the sheet (11).

3. The finger sheath of tampon as claimed in claim 2, **characterized in that** the outer edge (11b, 51b) of the sheet (11, 51) forms a round corner adjacent to the posterior edge (11d, 51d) of the sheet (11, 51).

4. The finger sheath of tampon as claimed in claim 1, **characterized in that** the sheet (11, 51) is made of degradable materials.

5. The finger sheath of tampon as claimed in claim 1, **characterized in that** the finger sheath of tampon is formed in a conical tubular shape, and wherein the entrance (13, 53) is larger than the opening (12, 52).

6. A telescopic catheter set, **characterized in** comprising:
an outer tube (1) having a sheet (11, 51) partially overlapped by curling, wherein a chamber (S) is formed inside the sheet (11, 51), wherein an inner edge (11a, 51a) of the sheet (11, 51) is not exposed and an outer edge (11b, 51b) of the sheet (11, 51) is exposed, wherein an anterior edge (11c, 51c) of the sheet (11, 51) circles to form an opening (12, 52), and wherein a posterior edge (11d, 51d) of the sheet (11, 51) circles to form an entrance (13, 53); and
an inner tube (2), wherein a first end (21) of the inner tube (2) is located inside the chamber (S) and is movable relative to the outer tube (1), and wherein a second end (22) of the inner tube (2) is located at the posterior edge (11d, 51d) of the entrance (13, 53).

7. The telescopic catheter set as claimed in claim 6, **characterized in that** the anterior edge (11c, 51c) of the sheet (11, 51) is shorter than the posterior edge (11d, 51d) of the sheet (11, 51), and wherein the outer edge (11b, 51b) of the sheet (11, 51) tiltedly connects to the anterior edge (11c, 51c) and the posterior edge (11d, 51d) of the sheet (11,51).

8. The telescopic catheter set as claimed in claim 6, **characterized in that** the outer edge (11b, 51b) of the sheet (11, 51) forms a round corner (14) adjacent to the posterior edge (11d, 51d) of the sheet (11, 51).

9. The telescopic catheter set as claimed in claim 6, **characterized in that** the outer tube (1) has an anti-slip portion positioned on an exposed surface of the sheet (11, 51), wherein the anti-slip portion is adjacent to the entrance (13, 53) and is away from the opening (12, 52).

10. The telescopic catheter set as claimed in claim 6, **characterized in that** the sheet (11, 51) is made of degradable materials.

11. The telescopic catheter set as claimed in any one of claims 6 to 10, **characterized in that** the inner tube (2) has a longitudinal ditch (23) extended from the first end (21) to the second end (22).

12. The telescopic catheter set as claimed in claim 11, **characterized in that** the inner tube (2) is crimped and thermoformed from a plate (24), and wherein the longitudinal ditch (23) is formed between a first lateral edge (24a) and a second lateral edge (24b) opposite to the first lateral edge (24a) of the plate (24).

13. The telescopic catheter set as claimed in claim 12, **characterized in** further comprising a connection belt (4), wherein a first end of the connection belt (4) connects to the inner edge (11a, 51a) of the sheet (11, 51), wherein a second end of the connection belt (4) connects to the first lateral edge (24a) of the plate (24), and wherein the connection belt (4) is coiled around a periphery of the inner tube (2) inside the chamber.

14. The telescopic catheter set as claimed in claim 13, **characterized in that** the first end of the connection belt (4) is adjacent to a posterior edge (11d, 51d) of the sheet (11, 51), and wherein the second end of the connection belt (4) is adjacent to an anterior end (24c) of the plate (24).

15. A catheter tampon, **characterized in** comprising:
a telescopic catheter set as claimed in any one of claims 6 to 14; and
a tampon (3, 5) having an absorption body (31) inside the chamber, with a string (32) positioned at end of the absorption body (31), and wherein the string passes through the inner tube (2) and through out of a second end (22) of the inner tube (2).
